(19) Europäisches Patentamt
European Patent Office
Office européen des brevets

(11) **EP 4 685 438 A1**

(12) **EUROPEAN PATENT APPLICATION**
published in accordance with Art. 153(4) EPC

(43) Date of publication:
**28.01.2026 Bulletin 2026/05**

(21) Application number: **24774711.6**

(22) Date of filing: **07.03.2024**

(51) International Patent Classification (IPC):
*G01D 3/032* (2006.01)    *A61B 5/242* (2021.01)
*G01R 33/02* (2006.01)

(52) Cooperative Patent Classification (CPC):
**A61B 5/242; G01D 3/032; G01R 33/02**

(86) International application number:
**PCT/JP2024/008841**

(87) International publication number:
**WO 2024/195572 (26.09.2024 Gazette 2024/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.03.2023 JP 2023045931**

(71) Applicant: **TDK Corporation
Tokyo 103-6128 (JP)**

(72) Inventor: **TERAZONO Yasushi
Tokyo 103-6128 (JP)**

(74) Representative: **Epping - Hermann - Fischer
Patentanwaltsgesellschaft mbH
Schloßschmidstraße 5
80639 München (DE)**

(54) **INFORMATION PROCESSING DEVICE, INFORMATION PROCESSING SYSTEM, INFORMATION PROCESSING METHOD, AND PROGRAM**

(57)     According to an embodiment, an information processing device includes an acquisition unit configured to acquire a multidimensional measurement signal obtained by measuring a target signal including a signal component having a repetitive structure and a signal processing unit configured to perform predetermined signal processing on the measurement signal acquired by the acquisition unit with a calculation model for performing signal separation using multidimensionality, the calculation model including a constraint that repetitive parts have the same value when a relative time from a repetition start time is identical.

FIG. 1

EP 4 685 438 A1

**Description**

TECHNICAL FIELD

The present disclosure relates to an information processing device, an information processing system, an information processing method, and a program.

BACKGROUND ART

Conventionally, in a process for reducing noise in multichannel measurement of repetitive events, methods are separately used as in a case where an arithmetic mean calculation method is used for repeatability and a factor analysis or blind signal separation method is used for multichannel capability.

For example, signal processing in which an arithmetic mean calculation process is performed after independent component analysis (ICA) is performed is disclosed in a biological signal measurement device described in Patent Document 1 (see Patent Document 1).

Citation List

Patent Document

Patent Document 1: Japanese Unexamined Patent Application, First Publication No. 2001-120511

SUMMARY OF INVENTION

Technical Problem

However, in the conventional technology, when an arithmetic mean calculation process is performed after factor analysis or blind signal separation, an estimated value of a signal does not approach a true value even if the number of additions is increased, and a signal waveform of an arithmetic mean calculation result tends to be distorted.

In contrast, when the factor analysis or blind signal separation is performed after the arithmetic mean calculation process, the number of samples in noise remaining after the arithmetic mean calculation process is significantly reduced (e.g., to (1/(Number of additions))) from the original data, which is disadvantageous for stable calculation of statistics. Moreover, in this case, the number of components asynchronous with a target signal (a signal of interest) are reduced by the arithmetic mean calculation process, making it difficult to separate the target signal.

The present disclosure has been made in consideration of the above circumstances, and an objective of the present disclosure is to provide an information processing device, an information processing system, an information processing method, and a program that enables analysis effectively using multidimensionality of a target signal and a repetitive structure of the target signal to be implemented.

Solution to Problem

According to an aspect, there is provided an information processing device including: an acquisition unit configured to acquire a multidimensional measurement signal obtained by measuring a target signal including a signal component having a repetitive structure; and a signal processing unit configured to perform predetermined signal processing on the measurement signal acquired by the acquisition unit with a calculation model for performing signal separation using multidimensionality, the calculation model including a constraint that repetitive parts have the same value when a relative time from a repetition start time is identical.

According to an aspect, there is provided an information processing system including: the information processing device; and a plurality of sensors configured to measure the measurement signal.

According to an aspect, there is provided an information processing method including: acquiring, by an acquisition unit of an information processing device, a multidimensional measurement signal obtained by measuring a target signal including a signal component having a repetitive structure; and performing, by a signal processing unit of the information processing device, predetermined signal processing on the measurement signal acquired by the acquisition unit with a calculation model for performing signal separation using multidimensionality, the calculation model including a constraint that repetitive parts have the same value when a relative time from a repetition start time is identical.

According to an aspect, there is provided a program for causing a computer to implement: an acquisition function of acquiring a multidimensional measurement signal obtained by measuring a target signal including a signal component having a repetitive structure; and a signal processing function of performing predetermined signal processing on the measurement signal acquired by the acquisition function with a calculation model for performing signal separation using multidimensionality, the calculation model including a constraint that repetitive parts have the same value when a relative time from a repetition start time is identical.

Advantageous Effects of Invention

In the information processing device, the information processing system, the information processing method, and the program according to the present disclosure, analysis effectively using multidimensionality of a target signal and a repetitive structure of the target signal can be implemented.

BRIEF DESCRIPTION OF DRAWINGS

[FIG. 1] A diagram showing an example of a configuration of an information processing system including an information processing device according to an embodiment.
[FIG. 2] A diagram showing an example of a configuration of a functional block of the information processing device according to an embodiment.
[FIG. 3] A diagram showing an example of a target signal according to an embodiment.
[FIG. 4] A diagram showing an example of sensor noise superimposed on a target signal according to an embodiment.
[FIG. 5] A diagram showing an example of an interference component superimposed on a target signal according to an embodiment.
[FIG. 6] A diagram showing an example of a measurement signal including a target signal according to an embodiment.
[FIG. 7] An explanatory diagram of an absolute time and an in-event time according to an embodiment.
[FIG. 8] A diagram showing an example of a low-signal period for a target signal according to an embodiment.
[FIG. 9] A diagram showing an example of an arithmetic mean calculation result according to a comparative example.
[FIG. 10] A diagram showing an example of a factor analysis result after an arithmetic mean calculation process according to a comparative example.
[FIG. 11] A diagram showing an example of a factor analysis result according to a comparative example.
[FIG. 12] A diagram showing an example of an arithmetic mean calculation result after factor analysis according to a comparative example.
[FIG. 13] A diagram showing an example of a relationship between the number of arithmetic mean calculation repetitions and an error from a true value according to a comparative example.

DESCRIPTION OF EMBODIMENTS

Hereinafter, embodiments of the present disclosure will be described with reference to the drawings.

[Information processing system]

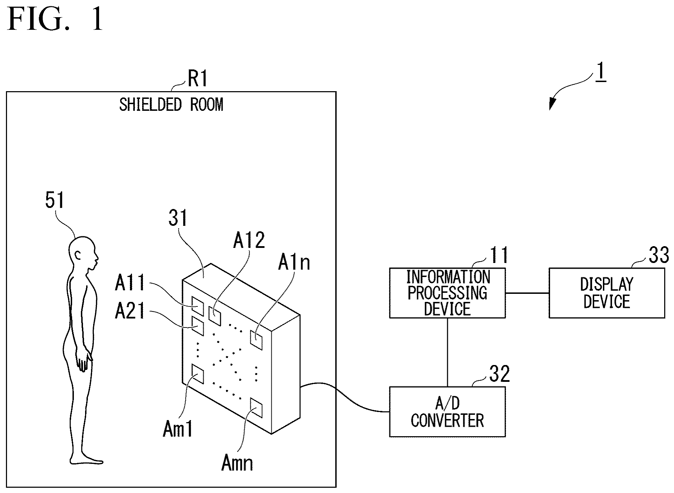

FIG. 1 is a diagram showing an example of a configuration of an information processing system 1 including an information processing device 11 according to an embodiment.
The information processing system 1 includes an information processing device 11, a sensor unit 31, an analog-to-digital (A/D) converter 32, a display device 33, and a shielded room R1.
Moreover, a human subject 51 is also shown in FIG. 1. In addition, a measurement target does not have to be a human.
Although the information processing system 1 includes at least the information processing device 11 in the present embodiment, some of the other components (the sensor unit 31, the A/D converter 32, the display device 33, and the shielded room R1) may not be included in the information processing system 1.
Moreover, the information processing system 1 may include constituent elements not shown in the present embodiment.
The sensor unit 31 includes $(m \times n)$ sensors Aij (i = 1 to m and j = 1 to n).
Here, m denotes an integer of 1 or more and n denotes an integer of 1 or more. In the present embodiment, one or both of m and n are integers of 2 or more and m and n have the same value. Thereby, in the present embodiment, $(m \times n)$ sensors Aij constitute a sensor array. In addition, m and n may have different values as another example.
However, when multidimensionality is assigned to a signal (data) by time-frequency conversion or the like to be described below, for example, a configuration in which both m and n are 1 may be used.

In the present embodiment, the sensor unit 31 is arranged inside the shielded room R1. The subject 51 undergoes a predetermined examination inside the shielded room R1.

In the present embodiment, a part of a body of the subject 51 (a diagnostic target part) is a target to be measured by the sensor Aij of the sensor unit 31.

In addition, instead of the word "measurement," words such as "observation" and "detection" may be used.

Although a case where the sensor unit 31 and the subject 51 are arranged inside the shielded room R1 and an examination is performed is shown in the present embodiment, such an aspect may be used when there is no problem even if one or both of the sensor unit 31 and the subject 51 are arranged outside the shielded room R1 as another example.

As an example, the sensor Aij is a magnetic sensor, the measurement target is the heart of the subject 51 (a magnetic field from the heart) and the shielded room R1 is a magnetically shielded room (MSR). As the magnetic sensor, for example, a magneto resistive (MR) sensor may be used.

In the present embodiment, the sensor unit 31 and the A/D converter 32 can communicate with each other by wire or wirelessly.

Moreover, the A/D converter 32 and the information processing device 11 can communicate with each other by wire or wirelessly.

Moreover, the information processing device 11 and the display device 33 can communicate with each other by wire or wirelessly.

Although the sensor unit 31, the A/D converter 32, and the information processing device 11 are separate in the present embodiment, for example, an A/D conversion function may be provided in the sensor unit 31 (e.g., each sensor Aij) or the information processing device 11. In this configuration, the A/D converter 32 may not be provided.

Although the case where the information processing device 11 and the display device 33 are separate is shown in the present embodiment, for example, the information processing device 11 and the display device 33 may be integrated.

<Outline of operation in information processing system>

The subject 51 is arranged at a position where the subject 51 can be measured by the sensor Aij of the sensor unit 31.

The sensor unit 31 detects a signal (e.g., on which noise is also superimposed) from the subject 51 with each of the multiple sensors Aij and outputs a plurality of detected signals (detected signals of the plurality of sensors Aij) to the A/D converter 32. In the present embodiment, the plurality of signals are analog signals.

The A/D converter 32 converts the plurality of signals input from the sensor unit 31 from analog signals into digital signals and inputs the plurality of converted signals (the digital signals) to the information processing device 11.

The information processing device 11 performs predetermined signal processing based on the plurality of signals (the digital signals) input from the A/D converter 32 and outputs information about a signal processing result to the display device 33. In the present embodiment, the signal processing in the information processing device 11 is performed on digital signals (digital data).

The display device 33 has a screen and displays and outputs information to be displayed on the screen on the basis of information input from the information processing device 11.

[Information processing device]

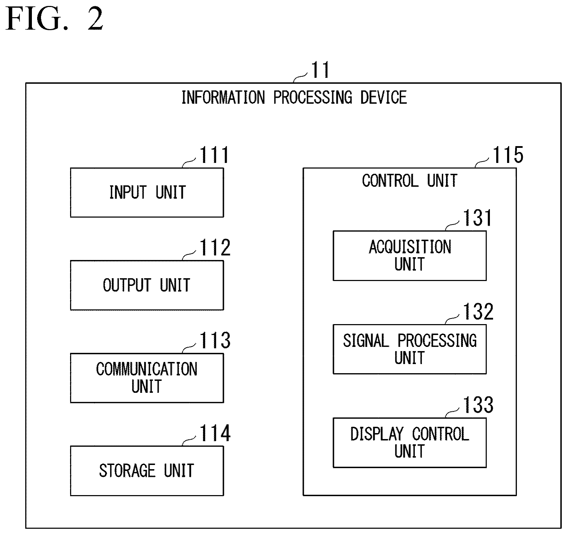

FIG. 2 is a diagram showing an example of a configuration of functional blocks of the information processing device 11 according to an embodiment.

The information processing device 11 includes an input unit 111, an output unit 112, a communication unit 113, a storage unit 114, and a control unit 115.

The control unit 115 includes an acquisition unit 131, a signal processing unit 132, and a display control unit 133.

The input unit 111 has a function of inputting information.

The input unit 111 may have, for example, an operation unit operated by a user, and may input information (including various types of instructions) corresponding to an operation performed on the operation unit.

Moreover, the input unit 111, for example, may be connected to an external device (e.g., a portable storage medium or the like) and input information from the device.

The output unit 112 has a function of outputting information.

For example, the output unit 112 may be connected to an external device (e.g., a portable storage medium or the like) and output information to the device.

In addition, the output unit 112 may output predetermined information by sound, light, or the like.

The communication unit 113 has a function of communicating with an external device (e.g., the A/D converter 32 or the display device 33). The communication includes transmission (output) and reception (input).

Here, in the present embodiment, for convenience of description, the input unit 111 and the output unit 112 are described separately from the communication unit 113. However, for example, the functions of the input unit 111 and the output unit 112 may be considered to include the function of the communication unit 113.

The storage unit 114 stores various types of information.

For example, the storage unit 114 may store a signal (signal data) input from the A/D converter 32, a signal (signal data) of a result of performing predetermined signal processing on the signal, or a signal (signal data) during the signal processing.

Moreover, the storage unit 114 may store any other information.

The control unit 115 performs various types of processes and control.

The acquisition unit 131 acquires data to be subjected to signal processing. In the present embodiment, the data is, for example, signal data input from the A/D converter 32. In addition, the acquisition unit 131 may acquire the data by reading the data from the storage unit 114 after the data is once stored in the storage unit 114.

The signal processing unit 132 performs predetermined signal processing (data processing) on the data acquired by the acquisition unit 131.

The display control unit 133 performs control for displaying information about a signal processing result or the like. The display control unit 133 performs control so that information to be displayed is output to the display device 33.

In the present embodiment, the information processing device 11 is configured as a computer.

The information processing device 11 has a processor such as a central processing unit (CPU) and performs various types of processes and control by executing a predetermined program by the processor. The program, for example, may be stored in the storage unit 114.

[Signal component of measurement signal and measurement signal]

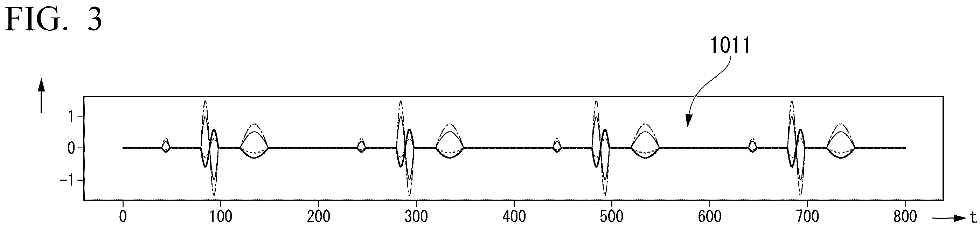

Examples of signal components of measurement signals and measurement signals will be described with reference to FIGS. 3 to 6.

In the graphs shown in FIGS. 3 to 6, the horizontal axis represents time (t) and the vertical axis represents a signal level.

<Target signal>

In the present embodiment, for convenience of description, a signal to be measured is referred to as a target signal. The target signal is a signal from a target to be measured.

In addition, such a signal may be referred to as any other name, for example, a signal of interest, an object signal, a desired signal, or the like.

FIG. 3 is a diagram showing an example of a target signal 1011 according to an embodiment.

In addition, in the example of FIG. 3, target signals included in signals (measurement signals) of a plurality of channels measured for the same target are collectively referred to as the target signal 1011.

Here, in the present embodiment, a plurality of measurement signals are obtained from the same target by a plurality of sensors Aij, and the measurement signals of the plurality of sensors Aij constitute the measurement signals of a plurality of channels.

That is, in the present embodiment, each sensor Aij corresponds to a channel.

In the present embodiment, the target signal 1011 has a structure in which the same (or similar) waveforms are repeated over time. That is, in the target signal 1011, the time periods in which the same (or similar) waveforms appear occur repeatedly at different times.

In this way, the target signal 1011 is a time-series signal including a signal component having repeatability.

In the example of FIG. 3, the target signal 1011 is a magneto-cardiogram (or an electrocardiogram) from the heart (heartbeat) of the subject 51.

In addition, a time period (repetition period) in which the same (or similar) waveforms are repeated in the target signal 1011 may be constant or may not be constant. When the repetition period is not constant, for example, the timing may be adjusted so that parts of the same (or similar) waveforms are treated as parts of the same (or similar) waveforms in signal processing, i.e., a timing adjustment may be made so that the relative times of the parts of the same (or similar) waveforms are aligned.

Here, a case where a signal from a living body (the subject 51) is used as the target signal is shown in the present embodiment. However, a signal from an object other than a living body may also be used. In other words, anything may be used as a measurement target.

Moreover, any sensor may be used as the sensor Aij, and, for example, a magnetic sensor, an electrical sensor, an optical sensor, an image sensor, an acceleration sensor, a sound sensor, or the like may be used.

Although a case where sensors of the same type (which may have individual differences) are used as the plurality of sensors Aij are shown in the present embodiment, different types of sensors may be used.

Although a case where the target signal itself has a component with a periodically repetitive structure is shown in the present embodiment, a signal of a result of measuring a signal that does not have a component of a periodically repetitive structure in a measurement system for repeatedly measuring the signal may be used as the target signal (here, for convenience of description, noise is ignored) as another example.

As a specific example, in a measurement system in which a predetermined object is placed on a turntable that rotates at a constant speed and the turntable is rotated, a signal of a result of periodically and repeatedly measuring a signal from an object using a sensor arranged at a fixed position may be used as a target signal (here, for convenience of description, noise is ignored).

<Sensor noise>

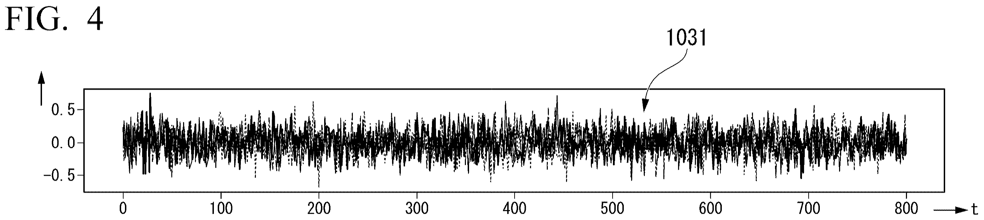

FIG. 4 is a diagram showing an example of sensor noise 1031 superimposed on the target signal 1011 according to the embodiment.

In the example of FIG. 4, sensor noise (a sensor noise signal) included in signals (measurement signals) of a plurality of channels measured for the same target is collectively referred to as sensor noise 1031.

The sensor noise 1031 is an example of noise.

<Interference component (interference signal)>

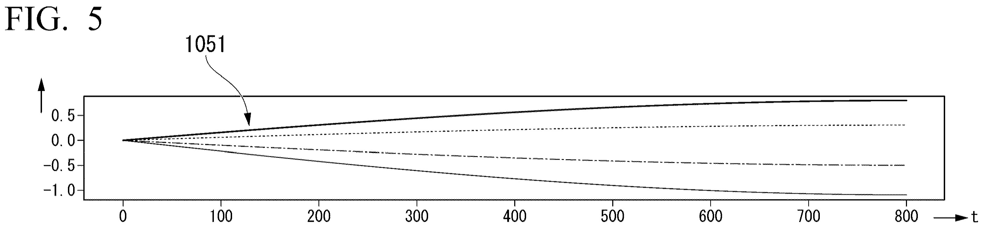

FIG. 5 is a diagram showing an example of an interference component 1051 superimposed on a target signal 1011 according to the embodiment.

In addition, in the example of FIG. 5, interference components (interference component signals) included in signals (measurement signals) of a plurality of channels measured for the same target will be collectively referred to as an interference component 1051.

Here, the interference component is, for example, a component that interferes with measurement due to a natural or artificial object located outside the sensor Aij.

As a specific example, when magnetism is measured, the interference component may be an influence of the earth's magnetic field (geomagnetic field) or an influence of magnetism generated from electronic devices.

The interference component 1051 is an example of noise.

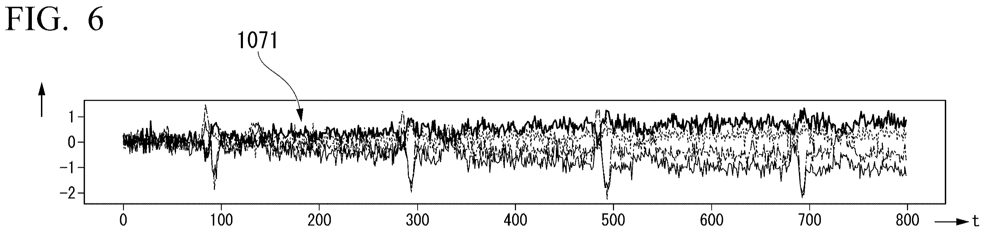

FIG. 6 is a diagram showing an example of a measurement signal 1071 including a target signal 1011 according to an embodiment.

In addition, in the example of FIG. 6, signals (measurement signals) of a plurality of channels measured for the same target will be collectively referred to as the measurement signal 1071.

Here, the measurement signal 1071 is a sum of the target signal 1011, the sensor noise 1031, and the interference component 1051.

That is, when the target signal 1011 is measured, the sensor noise 1031 and the interference component 1051 are superimposed on the target signal 1011.

Also, the measurement signal 1071 is a time-series signal on which a repetitive signal component is superimposed.

For this reason, a process for extracting only the component of the target signal 1011 by performing signal processing on the measurement signal 1071 is required.

<Multidimensionality>

In the present embodiment, multidimensionality refers to a case where there are a plurality of signals (signal data items) per sample (timing).

In the present embodiment, a plurality of sensors Aij arranged at different positions are used to measure a plurality of channels (multichannel measurement), thereby obtaining multidimensionality of the signal (multidimensionality of the signal data) for the target signal 1011.

Although the plurality of sensors Aij are arranged at different positions in the present embodiment, the plurality of sensors Aij are located within a range where they can receive the same target signal.

As another example, the multidimensionality of a signal (the multidimensionality of signal data) may be implemented by obtaining a plurality of signals from a signal obtained by measuring one channel (a single channel).

Moreover, the multidimensionality of a larger number of signals (the multidimensionality of signal data) may be implemented by obtaining a plurality of signals from each of two or more signals obtained by measuring a plurality of channels.

Here, a method for obtaining a plurality of signals from one signal is not particularly limited, and for example, a method using time-frequency transformation may be used.

In the method using time-frequency transformation, one signal is separated into signals of a plurality of frequency band components. Also, a multidimensional signal is implemented by treating a signal of one frequency band as one channel.

As another example, a multidimensional image based on the arrangement of pixels in the vertical and horizontal directions can be created.

That is, for a plurality of pixels arranged vertically and horizontally, the plurality of pixels arranged in the vertical direction can be treated as a plurality of channels (multichannel) and the plurality of pixels arranged in the horizontal direction can be treated as a plurality of samples (e.g., time-series data).

In contrast, the plurality of pixels arrayed in the vertical direction can be treated as a plurality of samples and the plurality of pixels arrayed in the horizontal direction can be treated as a plurality of channels.

As another example, a multidimensional image based on color components of an image can be created.

That is, when each pixel has a plurality of color components, the plurality of color components can be treated as a plurality of channels (multichannel) and the plurality of pixels can be treated as a plurality of samples (e.g., time-series data).

As an example, when pixels have color components R (red), G (green), and B (blue), RGB can be treated as three channels and the total number of pixels can be treated as the total number of samples.

[Absolute time and in-event time]

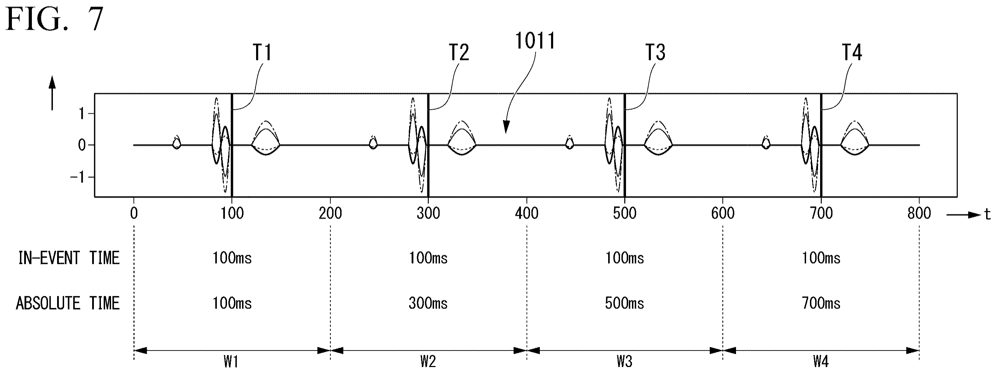

FIG. 7 is an explanatory diagram of an absolute time and an in-event time according to an embodiment.

In the graph shown in FIG. 7, the horizontal axis represents time (t) and the vertical axis represents a signal level.

In the graph, the same target signal 1011 is shown as in FIG. 3.

Moreover, in the graph, periods W1 to W4 of a plurality of events in which the same (or similar) waveform is periodically repeated, and in-event times T1 to T4, which are predetermined times within each event, are shown with respect to the target signal 1011.

Although four events are shown in the example of FIG. 7, if the target signal is also measured at the previous and subsequent times, there are also events at these times.

Here, in the present embodiment, for convenience of description, when the same (or similar) waveform is repeated in the target signal 1011, an event of one such waveform will be referred to as an event.

In addition, an event may be referred to as any other name.

Moreover, a period of the event may also be referred to as any other name, for example, an epoch or the like.

In the example of FIG. 7, the same (or similar) waveform of the target signal 1011 is repeated at intervals of 200 ms.

Each event has a start time of 0 ms and an end time of 200 ms (period W1), a start time of 200 ms and an end time of 400 ms (period W2), a start time of 400 ms and an end time of 600 ms (period W3), and a start time of 600 ms and an end time of 800 ms (period W4).

In addition, in the present embodiment, for simplicity of description, a case where the target signal 1011 has only a time (a period) belonging to an event is shown. However, the target signal 1011 may have a time (period) that does not belong to an event as another example. In this case, for example, the time that does not belong to the event may be excluded (i.e., only the time belonging to the event may be extracted) and signal processing may be performed.

For example, when the time period (repetition period) at which the same (or similar) waveforms repeat in the target signal 1011 is not uniform, the relative times of parts of the same (or similar) waveforms may be aligned in signal processing by excluding time that does not belong to an event (i.e., by extracting only the time that belongs to an event).

Here, in the present embodiment, a timing in the time (t) that elapses continuously regardless of an event will be referred to as an absolute time. In the present embodiment, locations where the type of time is specified will be described using the absolute time.

Moreover, in the present embodiment, a timing in the time that elapses when the start time of each event (the start time of each of periods W1 to W4) is considered to be 0 will be referred to as the in-event time.

In the example of FIG. 7, at an absolute time of 100 ms, the in-event time T1 in an event in period W1 is 100 ms.

Moreover, at an absolute time of 300 ms, the in-event time T2 in an event in period W2 is 100 ms.

Moreover, at an absolute time of 500 ms, the in-event time T3 in an event in period W3 is 100 ms.

Moreover, at an absolute time of 700 ms, the in-event time T4 in an event in period W4 is 100 ms.

In this way, the same value (0 ms to 200 ms in the example of FIG. 7) is used for the in-event time for the same (or similar) waveform.

In addition, the in-event time can be expressed as a function of the absolute time.

[Example of signal processing]

<Example of calculation model>

In the present embodiment, a calculation model shown in Eq. (1) is established to perform statistical estimation of A, Z, and N.

In this calculation model, for example, a probability is calculated according to the probability model, and parameters can be estimated by a likelihood estimation (expectation maximization (EM) or variational Bayes EM (VBEM) algorithm or the like.

[Math. 1]

$$X[t] = A*Z[tr]+N[t],$$

where * denotes the product of a matrix and a vector.

.. (1)

Here, in the normal factor analysis, the location of Z[tr] in Eq. (1) is roughly expressed as Z[t].

In Eq. (1), X[t], A, Z[tr], and N[t] each denote a matrix. In the present embodiment, X[t], Z[tr], and N[t] each denote a vector.

[t] denotes a value at the absolute time (t).
[tr] denotes a value at the in-event time (tr). The in-event time (tr) of each event denotes the time (relative time) elapsed from the start time (the absolute time t) of the event.
X[t] denotes a measurement signal at the absolute time.

A denotes a factor loading matrix for Z[t] when applied to factorization and denotes a value for describing an interchannel correlation by combining the activities of factors and giving a combining result to each sensor (the sensor Aij in the present embodiment).

Z[tr] denotes contracted components (factors) of the target signal when applied to factor analysis and is expressed in in-event time in the present embodiment. According to the term Z[tr], a term of mean estimation serving as the aggregate estimation is incorporated into the calculation model.

N[t] denotes noise at the absolute time.

In the example of FIG. 7, when the absolute time goes from 0 ms to 800 ms, the in-event time repeats four times from 0 ms to 200 ms.

In this case, X[t] and N[t] may vary when the absolute time goes from 0 ms to 800 ms.

In contrast, Z[tr] repeats the same value four times at intervals of 200 ms when the absolute time goes from 0 ms to 800 ms.

As a specific example, when t = 0 ms to 200 ms, tr = 0 ms to 200 ms. When t = 200 ms to 400 ms, tr = 0 ms to 200 ms. When t = 400 ms to 600 ms, tr = 0 ms to 200 ms. When t = 600 ms to 800 ms, tr = 0 ms to 200 ms.

In this way, in Eq. (1), the computation model includes a constraint that Z[tr] is the same when the in-event time (relative time) of each event having a repetitive structure is the same. In other words, Eq. (1) includes a condition that the repetitive signal components are made the same when factor analysis is performed.

Eq. (1) enables factor analysis corresponding to a repetitive structure, such that, for example, an effect similar to that of performing factor analysis and arithmetic mean calculation simultaneously is obtained.

In other words, in Eq. (1), an effect similar to that of adding a plurality of repetitive structures of signals having the same (or similar) waveforms according to the term Z[tr] is obtained.

<Reflection of absolute time to signal>

In the present embodiment, after a representative waveform of a repeated event is obtained by the calculation model of Eq. (1), the parameters of the calculation model determined at that time may be used to retroactively estimate the signal components of each event. The representative waveform is a representative waveform of the repeated part obtained by Eq. (1) using data of a plurality of events.

Eq. (2) that can be used for such estimation is shown.

In addition, for example, the start time of the event may be re-estimated from the signal components of the event estimated retroactively and analyzed again. Moreover, for example, this series of processes may be repeated any number of times (a desired number of times) or until the results converge.

[Math. 2]

$$Z[t] = A^{\wedge}T * (A * A^{\wedge}T + Phi)^{\wedge}(-1) * X[t],$$

where ^T denotes the transpose of a matrix,

^(-1) denotes an inverse matrix, and
* denotes the product of a matrix and a vector.
.. (2)

Here, A denotes a factor loading matrix independent of time, and is obtained, for example, by Eq. (1).

In Eq. (2), Z[t], A, Phi, and X[t] each denote a matrix. Among these, Z[t] and X[t] each denote a vector in the present embodiment.

Phi denotes a noise variance-covariance matrix, and is independent of time, and is obtained, for example, by Eq. (1).

[t] denotes a value at the absolute time (t).
X[t] denotes a measurement signal at the absolute time.
Z[t] denotes a contracted component (factor) of the target signal at the absolute time.

Z(t) at each absolute time (t) can be estimated by Eq. (2). Also, A*Z[t], which denotes a signal at the absolute time (t), can be obtained.

<Example of Bayes factor analysis>

In the present embodiment, the calculation model shown in Eq. (1) is based on Bayes factor analysis (BFA).

The target signal is a signal that has repeatability and has an inter-channel correlation (an inter-dimensional correlation).

Noise is not repetitive and does not have an inter-channel correlation.

<Another example of factor analysis>

As another example of factor analysis, the calculation model shown in Eq. (3) may be used.
[Math. 3]

$$X[t] = A * Z[tr] + B * Y[t] + N[t],$$

where * denotes the product of a matrix and a vector.

.. (3)

In Eq. (3), X[t], A, Z[tr], B, Y[t], and N[t] each denote a matrix. Among these, X[t], Z[tr], Y[t], and N[t] each denote a vector in the present embodiment.

Here, Y[t] denotes a contracted component (factor) of an interference component (an interference signal).

B denotes a factor loading matrix for Y[t], where [t] denotes a value at the absolute time (t).

X[t] denotes a measurement signal at the absolute time.

Z[tr] denotes a contracted component (factor) of the target signal at the in-event time.

A denotes a factor loading matrix for Z[tr].

Also, N[t] denotes a noise component other than B*Y[t] based on the interference component at the absolute time.

The target signal is a repetitive signal and has an inter-channel correlation (an inter-dimensional correlation).

The noise is not repetitive and does not have the inter-channel correlation. The interference component is a signal that does not have repeatability and has an inter-channel correlation.

An example of the interference component is a geomagnetic field or the like.

<Example of signal separation method>

In the present embodiment, a signal is separated into a plurality of one-dimensional components or multi-dimensional components using information about an inter-channel correlation or the like according to a signal separation method that utilizes multidimensionality.

In the present embodiment, a case where a model of factor analysis (FA) method is used as the signal separation method is shown.

Here, as the signal separation method, for example, a blind signal separation method may be used.

As the blind signal separation method, for example, a principal component analysis (PCA), independent component analysis (ICA), intelligent video analytics (IVA), and the like may be used.

<Example of signal processing considering low-signal period>

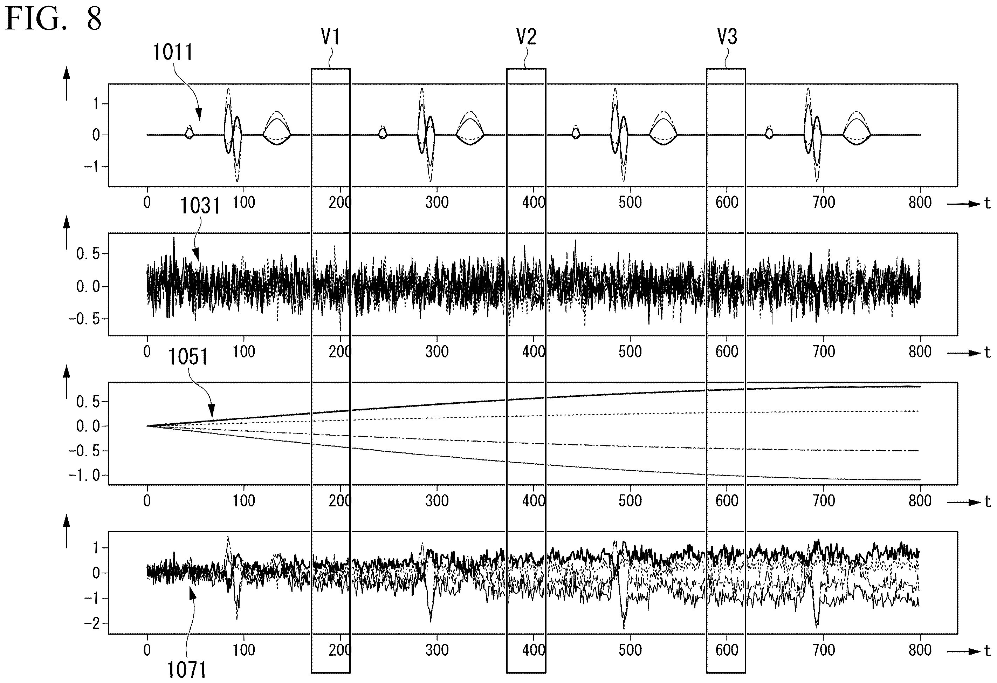

FIG. 8 is a diagram showing an example of low-signal periods V1 to V3 for a target signal 1011 according to an embodiment.

In the graph shown in FIG. 8, the horizontal axis represents time (t) and the vertical axis represents each signal level.

In the graph, the same target signal 1011 as shown in FIG. 3, the same sensor noise 1031 as shown in FIG. 4, the same interference component 1051 as shown in FIG. 5, and the same measurement signal 1071 as shown in FIG. 7 are shown.

Moreover, in the graph, low-signal periods (low-signal segments) V1 to V3 during which the signal level of the target signal 1011 is low are shown.

In the example of FIG. 8, each event has one of the low-signal periods V1 to V3.

Here, the low-signal periods V1 to V3 are periods in which the absolute value of the signal level (the absolute value of the signal value) of the target signal 1011 is less than or equal to a predetermined threshold value.

The threshold value may be any value (a value greater than or equal to 0 in the present embodiment), and may be, for example, a value close to zero.

As an example, when the target signal 1011 is an ideal signal (a signal in which noise is not completely present), the periods in which the signal level of the target signal 1011 is zero may be the low-signal periods V1 to V3.

In addition, periods other than the low-signal periods V1 to V3 may be referred to as, for example, non-low-signal periods (non-low signal segments).

Moreover, instead of the aspect in which the absolute value of the signal level is less than or equal to a predetermined threshold value, an aspect in which the absolute value of the signal level is less than a predetermined threshold value may be used.

The low-signal periods V1 to V3 may be arranged in the same position (the in-event time) for each event (i.e., may be arranged periodically) or may be located in a different position (the in-event time) for each event.

For example, when the period during which the absolute value of the signal level is less than or equal to a predetermined threshold differs according to each event, a common period for all events (a common portion during which the absolute value of the signal level is less than or equal to a predetermined threshold) may be set as the low-signal period or a different period for each event (a period during which the absolute value of the signal level is less than or equal to a predetermined threshold for each event) may be set as the low-signal period.

The signal (signal data) in the low-signal periods V1 to V3, for example, may be used as it is or may be removed from the signal itself (signal data itself) and may not be used or may be replaced with a signal (signal data) of a predetermined value (e.g., zero).

In this way, the signal in the period in which the target signal is not significantly present may be used to estimate the sensor noise or interference component.

Here, when the signal values (signal components) in the low-signal periods V1 to V3 are replaced with zero, it is assumed that there are only one or both of the value B*Y[t] based on the interference component and the value N[t] based on the noise, and this can be used to estimate the statistical characteristics thereof.

For example, statistical estimation may be performed using different calculation equations for the outside and inside of the low-signal periods V1 to V3. Even if the calculation equations are classified as cases in this way, calculations may be performed simultaneously for the entire signal (all signal data).

As a specific example, if Eq. (1) is used outside the low-signal periods V1 to V3 and Z (the in-event time) = 0 is set inside the low-signal periods V1 to V3, Eq. (4) is used inside the low-signal periods V1 to V3.
[Math. 4]

$$X[t] = N[t]$$

$$.. (4)$$

As another specific example, if Eq. (3) is used outside the low-signal periods V1 to V3 and Z (the in-event time) = 0 is set inside the low-signal periods V1 to V3, Eq. (5) is used inside the low-signal periods V1 to V3.
[Math. 5]

$$X[t] = B*Y[t]+N[t],$$

where * denotes the product of a matrix and a vector.

$$.. (5)$$

The low-signal period is a name for explanation and may be referred to as any other name.
For example, when a signal from a biological activity, such as a signal from a human heart (heartbeat), is measured, the low-signal period may be referred to as a non-activity period or the like.

<Example of signal processing considering differences in repetitive components>

Differences may occur for a plurality of events even in the signal components (repetitive components) of the repetitive structure of the target signal.
Therefore, an example of a calculation model in which the differences in such repetitive components are incorporated into the calculation model is shown in Eq. (6).
[Math. 6]

$$X[t] = A*(Z[tr]+V[t])+B*Y[t]+N[t],$$

where * denotes the product of a matrix and a vector.

$$.. (6)$$

Here, V[t] denotes a difference between repetitive structures of Z[tr] (factor).

[t] denotes a value at the absolute time (t).
[tr] denotes a value at the in-event time.

In addition, symbols identical to those in Eq. (3) denote the same things.
Here, V[t] is different from Z[tr] (factor) having a repetitive structure in that V[t] has no repetitive structure, the V[t] is different from Y[t] projected onto the data by B in that a composition matrix is different, and V[t] is different from N[t] that does not an inter-channel correlation in that there is a correlation between sensor channels.
By using Eq. (6), for example, a calculation result (the accuracy of the analysis result) can be improved compared to the case where the term V[t] of the difference between the repetitive components is not used.
Moreover, it is possible to ascertain a target-signal difference (a variation component) in each event using Eq. (6).
In addition, this may be applied to any case where a difference may occur in the repetitive component of the target signal, for example, when the heart (heartbeat) contains an arrhythmia component.
In this way, when signal processing is performed on a target signal whose repetitive component is generally the same each time but sometimes different, it is possible to clearly treat and extract a difference component between the repetitive components as a difference (V[t]) instead of treating it as noise.

[Signal processing according to comparative example]

In general, when noise other than a target signal is not synchronized with a repetitive structure of the target signal, an arithmetic mean is taken by adjusting a timing in synchronization with a part of the repetitive structure, such that noise that is a part of each fluctuation is reduced and the target signal to be reproduced each time is estimated. In other words, in the arithmetic mean calculation process, in accordance with a timing for each epoch, noise that is not related to the repetitive structure of the epoch is reduced by calculating an arithmetic mean of signals (signal data items) of a plurality of epochs. In summary, noise is reduced to approximately $(1/\sqrt{Q})$ by Q arithmetic mean calculations (Q is an integer of 2 or more).

Moreover, in general, factor analysis reduces (e.g., removes) asynchronous components between channels as components that cannot be explained by a common factor. For example, the factor analysis reduces noise in multi-dimensional data by decomposing into one-dimensional components that are uncorrelated (PCA) or independent (ICA) and selecting them or by decomposing into components that are correlated and components that are uncorrelated with each other.

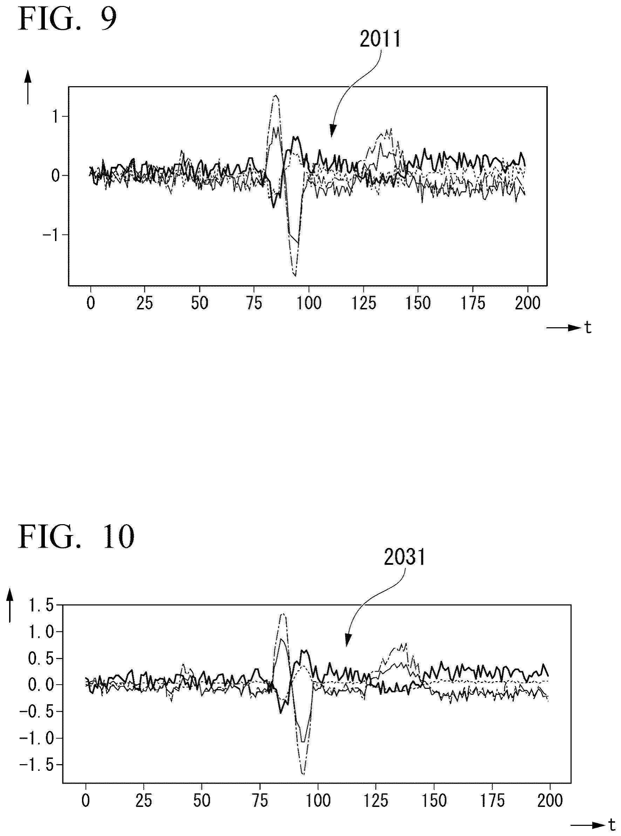

Signal processing according to a comparative example will be described with reference to FIGS. 9 to 13.

A target of signal processing according to the comparative example is a measurement signal similar to the measurement signal 1071 shown in FIG. 6.

In the graphs shown in FIGS. 9 to 12, the horizontal axis represents time (t) and the vertical axis represents a signal level.

<Example of signal processing in which factor analysis is performed after arithmetic mean calculation>

FIG. 9 is a diagram showing an example of an arithmetic mean calculation result 2011 according to a comparative example.

In the example of FIG. 9, the arithmetic mean calculation result 2011 is a result of calculating an arithmetic mean of the waveforms of four events in a measurement signal.

In arithmetic mean calculation, a waveform is obtained as a result of averaging single waveforms in events with respect to a plurality of events.

FIG. 10 is a diagram showing an example of a factor analysis result 2031 after arithmetic mean calculation according to a comparative example.

In the example of FIG. 10, the factor analysis result 2031 is a result of performing factor analysis on the arithmetic mean calculation result 2011 shown in FIG. 9, i.e., a result of performing factor analysis after arithmetic mean calculation on the measurement signal.

<Example of signal processing in which arithmetic mean calculation is performed after factor analysis>

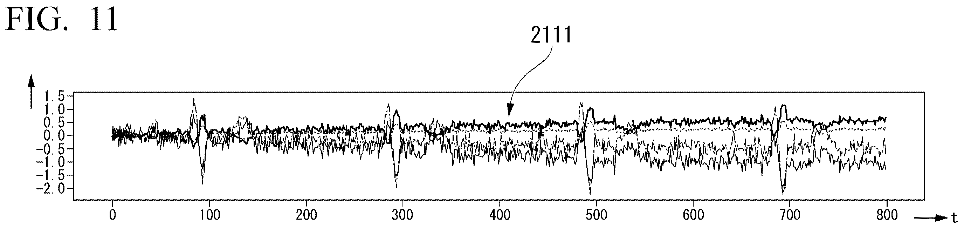

FIG. 11 is a diagram showing an example of a factor analysis result 2111 according to a comparative example.

In the example of FIG. 11, the factor analysis result 2111 is a result of performing factor analysis on a measurement signal.

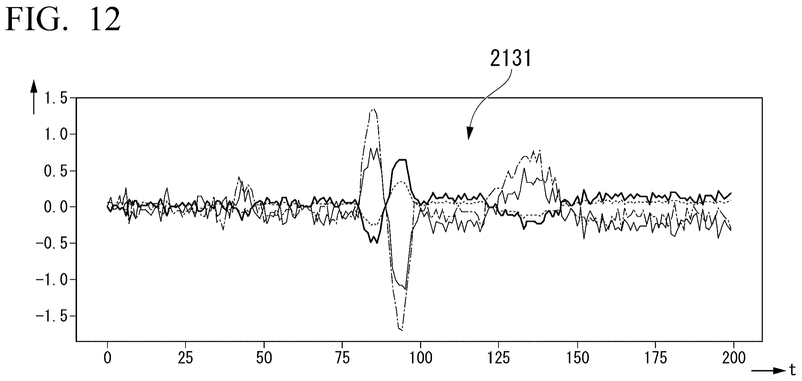

FIG. 12 is a diagram showing an example of an arithmetic mean calculation result 2131 after factor analysis according to a comparative example.

The example of FIG. 12 shows a result of calculating an arithmetic mean of the waveforms of four events for the factor analysis result 2111 shown in FIG. 11, i.e., an arithmetic mean calculation result after factor analysis is performed on the measurement signal.

<Number of arithmetic mean calculation repetitions>

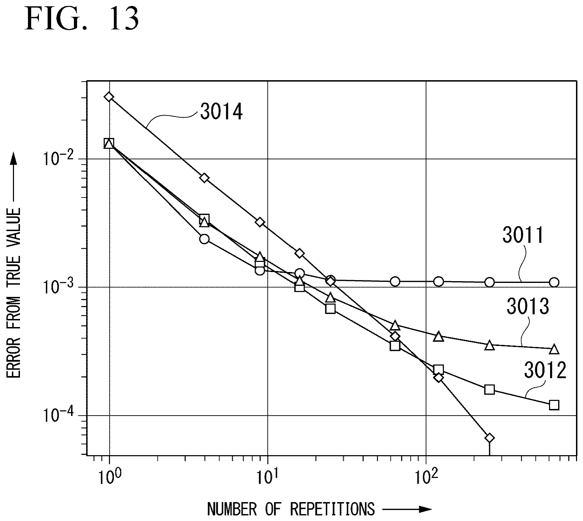

FIG. 13 is a diagram showing an example of a relationship between the number of arithmetic mean calculation repetitions and an error from a true value according to the comparative example.

In the graph shown in FIG. 13, the horizontal axis represents the number of arithmetic mean calculation repetitions and the vertical axis represents the error from the true value.

In the graph, characteristics 3011 to 3014 are shown.

The characteristic 3011 indicates a result of performing blind signal separation across all epochs and calculating an arithmetic mean from outputs thereof. Here, matrix A at the time of this calculation is common to all epochs.

The characteristic 3012 indicates a result of a case where the blind signal separation is performed after arithmetic mean calculation.

The characteristic 3013 indicates a result of performing the blind signal separation separately for each epoch and calculating an arithmetic mean from outputs thereof. Here, matrix A used in this calculation is different for each epoch.

The characteristic 3014 indicates a result of a case where only the arithmetic mean calculation is performed.

As a case where arithmetic mean calculation and signal separation are performed separately, a case where the arithmetic mean calculation is performed after the signal separation (the characteristic 3011) is relatively effective when the number of repetitions is very small, but the true value tends not to be approached even if the number of repetitions is increased.

As a case where arithmetic mean calculation and signal separation are performed separately, a case where the signal separation is performed after arithmetic mean calculation (the characteristic 3012) is effective up to a relatively large number of repetitions, but the true value tends not to be approached even if the number of repetitions is ultimately increased.

When signal separation is performed after arithmetic mean calculation (the characteristic 3013), the characteristics tend to be somewhere between these characteristics (the characteristics 3011 and 3012).

In the case of only the arithmetic mean calculation (the characteristic 3014), the true value tends to be approached more reliably as the number of repetitions increases.

<Comparison between embodiment and comparative example>

As a comparative example, when factor analysis is performed after arithmetic mean calculation, the number of samples in the noise remaining after arithmetic mean calculation is significantly reduced compared to the original data (which is (1/(Number of additions))), which reduces the accuracy of statistical calculation. Moreover, in this case, some components are lost due to arithmetic mean calculation, making it difficult to separate the target signal from the noise.

Moreover, as a comparative example, when arithmetic mean calculation is performed after factor analysis, it is thought that noise can be removed from the signal before addition (signal data). However, in practice, there is statistical inconsistency of estimation in which the true value is not approached when the arithmetic mean calculation is performed for the signals obtained through factor analysis.

In this way, when signal processing is performed by dividing the signal processing into two steps, i.e., arithmetic mean calculation and factor analysis, it is necessary to handle components to be reduced in subsequent-stage processing in previous-stage processing and it is easy to deviate from the optimal treatment. Moreover, in this case, in the subsequent-stage processing, it is not possible to utilize the information components lost in the previous-stage processing.

In other words, the signal processing according to this comparative example is inconsistent as statistical estimation and all information cannot be utilized efficiently.

To solve this problem of the comparative example, repeatability and multidimensionality are truly used simultaneously to implement statistically better estimation in the present embodiment. Thereby, in the present embodiment, signal processing with high noise removal performance and high signal extraction performance is implemented.

In the present embodiment, a signal (signal data) of a multichannel measurement result, a multidimensional signal (signal data) after time-frequency conversion, or the like is targeted. Also, in the present embodiment, the separation of the target signal from the noise component is attempted by largely utilizing (for example, fully utilizing) the fact that the target signal component is different from the noise component in that the target signal component is repeatable and correlated between dimensions (between sensors in the present embodiment).

As described above, the information processing system 1 according to the present embodiment can implement analysis that effectively utilizes the multidimensionality of a target signal and a repetitive structure of the target signal in the information processing device 11.

In the present embodiment, for example, the noise removal performance of a target signal having a repetitive structure can be improved in the information processing device 11.

In the present embodiment, for example, by incorporating the characteristics of the repetitive structure of the target signal into a calculation model of factor analysis or blind signal separation, the problem of the order between the arithmetic mean calculation process and the multidimensional signal processing according to the comparative example does not occur.

That is, in the present embodiment, for example, signal processing can be performed using information (statistical information) of all target signals of all dimensions at the same time, and noise removal performance can be improved compared to the comparative example.

Here, a case where predetermined signal processing is performed on a target signal in the information processing device 11 has been described in the present embodiment. However, for example, predetermined pre-processing may be performed on the target signal before the signal processing, predetermined post-processing may be performed on a signal processing result after the signal processing, or both the pre-processing and the post-processing may be performed.

For example, the pre-processing may include various types of filtering processes.

For example, the post-processing may include a process of assigning a predetermined offset to a signal (signal data) and the like.

In the signal processing according to the present embodiment, when a signal separation method using multidimensionality is applied to a time-series signal (signal data) on which a signal component having repeatability is superimposed, a constraint that the signal component having repeatability has the same value when the relative time from the start time of each repetition is the same is included in the calculation model, and the multidimensionality and repeatability are simultaneously used to separate the signal having repeatability from components other than the signal (other components).

Therefore, in the signal processing according to the present embodiment, the accuracy of separating the signal component can be improved by simultaneously using the multidimensionality and the repeatability, and the extraction accuracy of the signal component and noise reduction performance can be improved.

Moreover, in the signal processing according to the present embodiment, because the signal processing is performed consistently with one calculation model, for example, the calculation result asymptotically approaches the true value by increasing the number of repetitive parts (the number of times corresponding to the number of additions).

In the signal processing according to the present embodiment, it is possible to implement multidimensionality by performing measurements on a plurality of channels.

Therefore, in the signal processing according to the present embodiment, for example, it is possible to perform signal separation by utilizing the characteristics of a multichannel sensor array.

In the signal processing according to the present embodiment, it is possible to implement multidimensionality by converting a signal (signal data) into a frequency domain and dividing the converted signal (signal data).

Therefore, in the signal processing according to the present embodiment, for example, even if single-channel measurement is performed, it is possible to perform signal separation using a characteristic difference for each frequency band.

In addition, both a multichannel measurement process and a process for classifying frequencies into frequency bands with respect to channels may be combined.

In the signal processing according to the present embodiment, for example, it is possible to recalculate the unique values for each repetition (the values of each repetition component that are not subject to the constraint that the values are the same when the relative time from the start time of each repetition is the same) before the signal processing according to the present embodiment is performed, using the parameters of the calculation model obtained by the signal processing as described above.

Therefore, in the signal processing according to the present embodiment, it is possible to feed back the knowledge obtained using the repeatability and re-estimate the signal values for each repetition (data that is not necessarily the same between a plurality of repetitions). Thereby, it is possible to improve the accuracy of the calculation results.

In the signal processing according to the present embodiment, for example, a calculation model having a term of a signal component having an inter-dimensional correlation and repeatability and a term of a signal component that does not have the inter-dimensional correlation can be used as the calculation model.

Therefore, in the signal processing according to the present embodiment, it is possible to use a calculation model called (target signal + uncorrelated noise). In addition, this calculation model corresponds to a model of so-called Bayes factor analysis.

In the signal processing according to the present embodiment, for example, a calculation model having a term of a signal component having an inter-dimensional correlation and repeatability, a term of a signal component having an inter-dimensional correlation without any repeatability, and a term of a signal component that does not have the inter-dimensional correlation can be used as the calculation model.

Therefore, in the signal processing according to the present embodiment, it is possible to use a calculation model of (target signal + interference component + uncorrelated noise).

In addition, in the present embodiment, either one of a calculation model of (target signal + uncorrelated noise) and a calculation model of (target signal + interference component + uncorrelated noise) may be used.

In the signal processing according to the present embodiment, for example, the use of multidimensionality can be achieved using an aspect based on a factor analysis model.

Therefore, in the signal processing according to the present embodiment, it is possible to implement separation of a signal having an inter-dimensional correlation and noise that does not have an inter-dimensional correlation.

In the signal processing according to the present embodiment, for example, the use of multidimensionality can be achieved using an aspect based on a blind signal separation model.

Therefore, in the signal processing according to the present embodiment, it is possible to implement separation of a signal having an inter-dimensional correlation and noise that does not have an inter-dimensional correlation.

As an example, when independent component analysis (ICA) is used, it is possible to decompose a signal into independent components, and set a specified number of components among the independent components as signal components having repeatability.

In addition, in the present embodiment, either one of a factor analysis model and a blind signal separation model may be used.

In the signal processing according to the present embodiment, for example, it is possible to improve the estimation accuracy of the entire signal processing by defining a segment (a non-low-signal segment) in which it is assumed that the signal component having repeatability (here, the absolute value) can take a value exceeding a predetermined threshold value (e.g., a non-zero value) and a segment (a low-signal segment) in which it is assumed that the signal component having repeatability (here, the absolute value) is always a value less than or equal to the threshold value (e.g., zero) and utilizing the signal (signal data) in the low-signal segment to estimate the characteristics of the signal component that does not have repeatability.

Therefore, in the signal processing according to the present embodiment, it is possible to improve the separation accuracy of the signal component having repeatability. In this way, it is possible to improve the accuracy of the calculation result.

That is, the estimation of the component having repeatability is implemented by separating the signal (signal data) into a component having repeatability and a component that does not have repeatability. At this time, it is possible to contribute to the estimation of the characteristics of the component that does not have repeatability in the time period in which the component having a repetitive structure does not appear as well as the time period in which the component having a repetitive structure appears. Thereby, the estimation accuracy of the characteristics of the component that does not have repeatability is improved. As a result, the separation accuracy of the component having repeatability is improved.

In addition, the distinction between these two segments (a low-signal segment and a non-low-signal segment) does not necessarily have to be used.

Also, a program for implementing the function of any constituent part of any device described above may be recorded on a computer-readable recording medium and the program may be read and executed by a computer system. Also, the "computer system" used here may include an operating system (OS) or hardware such as peripheral devices. Also, the "computer-readable recording medium" refers to a flexible disk, a magneto-optical disc, a read-only memory (ROM), a portable medium such as a compact disc-ROM (CD-ROM), or a storage device such as a hard disk embedded in the computer system. Furthermore, the "computer-readable recording medium" is assumed to include a medium that holds a program for a certain period of time, such as a volatile memory inside a computer system serving as a server or a client when the program is transmitted via a network such as the Internet or a communication circuit such as a telephone circuit. For example, the volatile memory may be a random-access memory (RAM). For example, the recording medium may be a non-transitory recording medium.

Moreover, the above-described program may be transmitted from a computer system storing the program in a storage device or the like to another computer system via a transmission medium or by transmission waves in a transmission medium. Here, the "transmission medium" for transmitting the program refers to a medium having a function of transmitting information, as in a network such as the Internet or a communication circuit such as a telephone circuit.

Moreover, the above-described program may be a program for implementing some of the above-described functions. Furthermore, the above-described program may be a so-called differential file capable of implementing the above-described function in combination with a program already recorded on the computer system. The differential file may be referred to as a differential program.

Moreover, the function of any constituent part of any device described above may be implemented by a processor. For example, each process in the embodiment may be implemented by a processor that operates on the basis of information of a program or the like and a computer-readable recording medium that stores information of a program or the like. Here, in the processor, for example, the function of each part may be implemented by individual hardware or the function of each part may be implemented by integrated hardware. For example, the processor may include hardware and the hardware may include at least one of a circuit that processes a digital signal and a circuit that processes an analog signal. For example, the processor may be configured using one or more circuit devices or/and one or more circuit elements mounted on a circuit board. An integrated circuit (IC) or the like may be used as the circuit device and a resistor, a capacitor, or the like may be used as the circuit element.

Here, the processor may be, for example, a CPU. However, the processor is not limited to the CPU and, for example, various types of processors such as a graphics processing unit (GPU) or a digital signal processor (DSP) may be used. Moreover, for example, the processor may be a hardware circuit based on an application-specific integrated circuit (ASIC). Moreover, the processor may include, for example, a plurality of CPUs, or may include a hardware circuit of a plurality of ASICs. Moreover, the processor may include, for example, a combination of a plurality of CPUs and a hardware circuit including a plurality of ASICs. Moreover, the processor may include, for example, one or more of an amplifier circuit and a filter circuit for processing an analog signal and the like.

Although embodiments of the present disclosure have been described in detail above with reference to the drawings, specific configurations are not limited to the embodiments and other designs and the like may also be included without departing from the scope of the present disclosure.

[Appendixes]

Hereinafter, (Configuration example 1) to (Configuration example 12) are shown.

(Configuration example 1) An information processing device including:

an acquisition unit configured to acquire a multidimensional measurement signal obtained by measuring a target signal including a signal component having a repetitive structure; and
a signal processing unit configured to perform predetermined signal processing on the measurement signal acquired by the acquisition unit with a calculation model for performing signal separation using multidimensionality, the calculation model including a constraint that repetitive parts have the same value when a relative time from a repetition start time is identical.

(Configuration example 2) The information processing device according to (Configuration example 1), wherein the multidimensional measurement signal is based on a result of measuring a predetermined signal in a plurality of channels.

(Configuration example 3) The information processing device according to (Configuration example 1) or (configuration example 2), wherein the multidimensional measurement signal is based on a result of dividing a predetermined signal into a plurality of frequency band signals.

(Configuration example 4) The information processing device according to any one of (Configuration example 1) to (Configuration example 3), wherein the signal processing unit calculates a part of each repetition to which the constraint is not applied on the basis of a signal processing result.

(Configuration example 5) The information processing device according to any one of (Configuration examples 1) to (Configuration example 4), wherein the calculation model includes a first signal component that has an inter-dimensional correlation and a repetitive structure and a second signal component that does not have the inter-dimensional correlation.

(Configuration example 6) The information processing device according to (Configuration example 5), wherein the calculation model further includes a third signal component that has an inter-dimensional correlation and does not have a repetitive structure.

(Configuration example 7) The information processing device according to any one of (Configuration example 1) to (Configuration example 6), wherein the calculation model is based on factor analysis with respect to multidimensionality.

(Configuration example 8) The information processing device according to any one of (Configuration example 1) to (Configuration example 6), wherein the calculation model is based on blind signal separation with respect to multidimensionality

(Configuration example 9) The information processing device according to any one of (Configuration example 1) to (Configuration example 8), wherein the signal processing unit performs the signal processing by setting a first segment in which an absolute value of the signal value is assumed to be a value less than or equal to a predetermined threshold value or a value less than the threshold value and a second segment in which the absolute value of the signal value is assumed to take other values, with respect to the signal component having the repetitive structure.

In the present embodiment, it is possible to provide an information processing system including an information processing device.

(Configuration example 10) An information processing system including:

the information processing device according to any one of (Configuration example 1) to (Configuration example 10); and
a plurality of sensors configured to measure the measurement signal.

In the present embodiment, it is also possible to provide an information processing method that is performed in an information processing device.

(Configuration example 11) An information processing method including:

acquiring, by an acquisition unit of an information processing device, a multidimensional measurement signal obtained by measuring a target signal including a signal component having a repetitive structure; and
performing, by a signal processing unit of the information processing device, predetermined signal processing on the measurement signal acquired by the acquisition unit with a calculation model for performing signal separation using multidimensionality, the calculation model including a constraint that repetitive parts have the same value when a relative time from a repetition start time is identical.

In the present embodiment, it is also possible to provide a computer-readable program (a computer program) constituting an information processing device.

(Configuration example 12) A program for causing a computer to implement:

an acquisition function of acquiring a multidimensional measurement signal obtained by measuring a target signal including a signal component having a repetitive structure; and
a signal processing function of performing predetermined signal processing on the measurement signal acquired by the acquisition function with a calculation model for performing signal separation using multidimensionality, the calculation model including a constraint that repetitive parts have the same value when a relative time from a repetition start time is identical.

REFERENCE SIGNS LIST

1 Information processing system
11 Information processing device
31 Sensor unit
32 A/D converter
33 Display device
51 Subject
111 Input unit
112 Output unit
113 Communication unit
114 Storage unit
115 Control unit
131 Acquisition unit
132 Signal processing unit
133 Display control unit
1011 Target signal
1031 Sensor noise
1051 Interference component
1071 Measurement signal
2011, 2131 Arithmetic mean calculation result
2031, 2111 Factor analysis result
3011 to 3014 Characteristic
Aij Sensor
T1 to T4 In-event times
V1 to V4 Low-signal period
W1 to W4 Period

**Claims**

1. An information processing device comprising:

   an acquisition unit configured to acquire a multidimensional measurement signal obtained by measuring a target signal including a signal component having a repetitive structure; and
   a signal processing unit configured to perform predetermined signal processing on the measurement signal acquired by the acquisition unit with a calculation model for performing signal separation using multidimensionality, the calculation model including a constraint that repetitive parts have the same value when a relative time from a repetition start time is identical.

2. The information processing device according to claim 1, wherein the multidimensional measurement signal is based on a result of measuring a predetermined signal in a plurality of channels.

3. The information processing device according to claim 1, wherein the multidimensional measurement signal is based on a result of dividing a predetermined signal into a plurality of frequency band signals.

4. The information processing device according to claim 1, wherein the signal processing unit calculates a part of each repetition to which the constraint is not applied on the basis of a signal processing result.

5. The information processing device according to claim 1, wherein the calculation model includes a first signal component that has an inter-dimensional correlation and a repetitive structure and a second signal component that does not have the inter-dimensional correlation.

6. The information processing device according to claim 5, wherein the calculation model further includes a third signal component that has an inter-dimensional correlation and does not have a repetitive structure.

7. The information processing device according to claim 1, wherein the calculation model is based on factor analysis with respect to multidimensionality.

8. The information processing device according to claim 1, wherein the calculation model is based on blind signal separation with respect to multidimensionality.

9. The information processing device according to claim 1, wherein the signal processing unit performs the signal processing by setting a first segment in which an absolute value of the signal value is assumed to be a value less than or equal to a predetermined threshold value or a value less than the threshold value and a second segment in which the absolute value of the signal value is assumed to take other values, with respect to the signal component having the repetitive structure.

10. An information processing system comprising:

   the information processing device according to any one of claims 1 to 9; and
   a plurality of sensors configured to measure the measurement signal.

11. An information processing method comprising:

   acquiring, by an acquisition unit of an information processing device, a multidimensional measurement signal obtained by measuring a target signal including a signal component having a repetitive structure; and
   performing, by a signal processing unit of the information processing device, predetermined signal processing on the measurement signal acquired by the acquisition unit with a calculation model for performing signal separation using multidimensionality, the calculation model including a constraint that repetitive parts have the same value when a relative time from a repetition start time is identical.

12. A program for causing a computer to implement:

   an acquisition function of acquiring a multidimensional measurement signal obtained by measuring a target signal including a signal component having a repetitive structure; and
   a signal processing function of performing predetermined signal processing on the measurement signal acquired by the acquisition function with a calculation model for performing signal separation using multidimensionality, the calculation model including a constraint that repetitive parts have the same value when a relative time from a repetition start time is identical.

# FIG. 1

# FIG. 2

INFORMATION PROCESSING DEVICE — 11

INPUT UNIT — 111

OUTPUT UNIT — 112

COMMUNICATION UNIT — 113

STORAGE UNIT — 114

CONTROL UNIT — 115

ACQUISITION UNIT — 131

SIGNAL PROCESSING UNIT — 132

DISPLAY CONTROL UNIT — 133

FIG. 3

FIG. 4

FIG. 5

FIG. 6

FIG. 7

FIG. 8

EP 4 685 438 A1

FIG. 9

2011

FIG. 10

2031

EP 4 685 438 A1

FIG. 11

FIG. 12

2131

FIG. 13

**EP 4 685 438 A1**

## INTERNATIONAL SEARCH REPORT

| International application No. |
|---|
| **PCT/JP2024/008841** |

| A. | CLASSIFICATION OF SUBJECT MATTER |
|---|---|

*G01D 3/032*(2006.01)i; *A61B 5/242*(2021.01)i; *G01R 33/02*(2006.01)i
FI: G01D3/032; A61B5/242; G01R33/02 R

According to International Patent Classification (IPC) or to both national classification and IPC

| B. | FIELDS SEARCHED |
|---|---|

Minimum documentation searched (classification system followed by classification symbols)

G01D3/00-3/036; A61B5/00-5/398; G01R33/00-33/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 6664568 B1 (ASAHI KASEI EMD CORPORATION) 13 March 2020 (2020-03-13) paragraphs [0019]-[0107], fig. 1-15 | 1-12 |
| A | JP 2006-317194 A (MITSUBISHI HEAVY INDUSTRIES, LTD.) 24 November 2006 (2006-11-24) paragraphs [0028]-[0062], fig. 1-13 | 1-12 |
| A | JP 2008-286561 A (KABUSHIKI KAISHA TOYOTA JIDOSHOKKI) 27 November 2008 (2008-11-27) paragraphs [0015]-[0042], fig. 1-5 | 1-12 |
| P, A | JP 2023-87434 A (TDK CORPORATION) 23 June 2023 (2023-06-23) paragraphs [0065]-[0130], fig. 3-15 | 1-12 |

☐ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

31

## INTERNATIONAL SEARCH REPORT
### Information on patent family members

International application No.

**PCT/JP2024/008841**

| Patent document cited in search report | | | Publication date (day/month/year) | Patent family member(s) | | | Publication date (day/month/year) |
|---|---|---|---|---|---|---|---|
| JP | 6664568 | B1 | 13 March 2020 | US | 2021/0161420 | A1 | |
| | | | | paragraphs [0042]-[0133], fig. 1-15 | | | |
| | | | | WO | 2020/040168 | A1 | |
| JP | 2006-317194 | A | 24 November 2006 | (Family: none) | | | |
| JP | 2008-286561 | A | 27 November 2008 | (Family: none) | | | |
| JP | 2023-87434 | A | 23 June 2023 | US | 2023/0188232 | A1 | |
| | | | | paragraphs [0149]-[0306], fig. 3-15 | | | |
| | | | | EP | 4194871 | A1 | |
| | | | | CN | 116263808 | A | |

Form PCT/ISA/210 (patent family annex) (July 2022)

## EP 4 685 438 A1

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001120511 A